(19) Europäisches Patentamt
European Patent Office
Office européen des brevets

(11) Publication number: **0 159 070 A1**

(12) # EUROPEAN PATENT APPLICATION

(21) Application number: 85200438.1

(22) Date of filing: 21.03.85

(51) Int. Cl.[4]: C 08 G 18/10
C 08 G 18/48, C 08 G 69/20

(30) Priority: 22.03.84 US 592082

(43) Date of publication of application:
23.10.85 Bulletin 85/43

(84) Designated Contracting States:
BE CH DE FR GB IT LI NL

(71) Applicant: STAMICARBON B.V.
LICENSING SUBSIDIARY OF DSM Postbus 53
NL-6160 AB Geleen(NL)

(72) Inventor: Ashida, Kaneyoshi
23560 East Newell Circle
Farmington Hills Michigan 48024(US)

(72) Inventor: Van der Loos, Jozef Lambertus M.
Rijksweg Zuid 146
NL-6134 AE Sittard(NL)

(74) Representative: van Leeuwen, Hendrik Bernardus et al,
Octrooibureau DSM Postbus 9
NL-6160 MA Geleen(NL)

(54) **N-substituted carbamoyl-lactam.**

(57) An N-substituted carbamoyl-lactam compound of the formula

```
R[-O-C-N-R'-(N-C-N-C=O)a]2
     ‖ |       ‖
     O H     H O  Y
```

wherein R is a radical derived from polytetramethyleneglycol $R(OH)_2$ and represents the radical remaining from the polytetramethylene oxide starting material used in preparing polytetramethyleneglycol and has an equivalent weight of 300-2500,

R' is an alkylene, cycloalkylene, arylene, alkarylene or an aralkylene group having 6-25 carbon atoms,

Y is a $C_3$-$C_{14}$ alkylene group, and

a has a value of at least 1.

This compound is suitable as an activator for the lactam polymerization, especially in RIM applications.

EP 0 159 070 A1

# N-SUBSTITUTED CARBAMOYL-LACTAM

## BACKGROUND OF THE INVENTION

The present invention relates to an N-substituted carbamoyl-lactam.

In the anionic polymerization of a lactam, especially caprolactam, an anionic polymerization catalyst and an activator (or promoter) are typically present. In the art various kinds of activators are disclosed.

One proposal for an activated anionic catalytic polymerization process is described in U.S. Patent 3,304,291. The activator used therein consists of an organic nitrogen compound having an at least 3 to 12 carbon radical being an N-substituted compound of at least one urea, thiourea or guanidine radical.

An improvement thereon for producing polylactams having a higher notch impact strength includes conducting the activated anionic catalyzed lactam polymerization process in the presence of a quantity of a polyether soluble in the molten lactam or the mixture of lactams being polymerized. The polyether is limited to at most about 25 % by weight of the quantity of the lactam to be polymerized, otherwise the resultant polylactams possess poor mechanical properties.

Another improved process for the anionic catalytic polymerization of lactams aided by one or more promoters described in U.S. patent No. 3,770,689 includes adding to the reaction mixture one or more polyether compounds having etherified hydroxyl groups which are soluble in the molten lactam or lactam mixture. Conventional promoters suitable for use therein include polymer chains permanently terminated on at least one end by a promoter function. Generally the promoter functional groups or substituents are similar to monomeric promoters such as acid-chloride groups, isocyanates, N-carbonyl-lactam groups, imide groups, N-carbonyl-sulfonamide groups, N-carbonyl-urea groups

and acid-anhydride groups.

U.S. Patent No. 3987033 describes a composition prepared by reacting an aromatic diisocyanate with a triprimary alcohol and subsequently reacting this product with a mixture of a hydroxy component such as a phenol.

U.S. Patent No. 4171305 describes pure cyrstals of ε-caprolactam diblocked ω,ω'-diisocyanato-1,3-dimethylbenzene used as a hardener of powder coating composition.

U.S. Patent No. 4211699 describes isocyanate adduct diols derived from an amino diol or a hydrazine diol and an organic diisocyanate and their use for the production of self-corsslinkable and/or self-crosslincked polyurethanes.

U.S. Patent No. 3,018,273 describes a process for polymerizing caprolactam in situ in the presence of an organomagnesium initiator compound and an N,N diacyl compound which is used as a promoter.

Preferably, the N,N diacyl compounds are N-substituted imides, such as cyclic imides of dicarboxylic acids, having molecular weights not exceeding 1000 and more preferably the molecular weights do not exceed 500 in order to preclude the presence of large inert groups in the promoters.

British patent No. 1,067,153 describes a process for preparing nylon-block-copolymers by anionically polymerizing caprolactam in the presence of an isocyanate capped polypropylene glycol and a potassium based catalyst. In this process a nylon block copolymer containing at least one polyether block is formed.

In the U.S. patents Nos. 3,862,262, 4,031,164, 4,034,015 and 4,223,112 various aspects of the preparation of nylon block copolymers from caprolactam in the presence of an acyl lactam activator are described.

U.S. patents Nos. 4,031,164 and 4,223,112 describe lactam-polyol-polyacyl-lactam-block terpolymers having a specified ratio of the various components. More particularly U.S. Patent No. 4,031,164 discloses the use of 18 to 90 % by weight of polyol blocks in the terpolymer.

U.S. patent No. 3,862,262 describes lactam-polyol-acyl-polylactam block-terpolymers, and U.S. patent No. 4,034,015 is directed to lactam polyol-polyacyllactam or lactam-polyol-acyl-poly-lactam block terpolymers having at least about 5 % ester end group termination.

The Reissue patent 30,371 describes the preparation of polyester-polyamide compounds by condensation of an alcohol and an acyl lactam in the presence of at least one of a metal or metal compound, the metal components thereof being selected from Group IA, IIA, IIB and IIIa of the Periodic Table.

Preparation of nylon compositions prepared by anionic polymerization of at least 75 % lactam with up to about 25 % of an epoxy component in the presence of a basic catalyst and promoter are disclosed in U.S. Patent No. 4,400,490. The promoters are those typically used in the anhydrous polymerization of lactams.

In Sibal et al, Designing Nylon 6 polymerization systems, presented in part at the 2nd International Conference on Reactive Polymer Processing, Pittsburg Pa Nov. 1982, suggests in connection with the anhydrous anionic polymerization of caprolactam to prepare a cocatalyst or initiator by reacting at 80 °C isocyanate with dried caprolactam. Initially the dried caprolactam may be heated and about 20 % thereof boiled off with a residue portion being reacted with an isocyanate, the isocyanate being obtained by slowly reacting 1 mole polypropylene glycol (M.W. 2000) with 2 moles hexamethylene diisocyanate. However, it is clear that further work is needed to determine processability of polylactams produced using this promoter.

The published (December 22, 1982) European patent Applications Nos. 67693 and 67694 decribe specific lactam compounds based on various kinds of hydroxy compounds. European Patent Applications 67694 and 67695 (published December 22, 1982) describe the use of these lactam compounds in the preparation of nylon.

The object of the invention is to provide an N-substituted carbamoyl-lactam compound which can be used as an activator in the preparation of nylon block copolymers, having improved physical properties.

The invention is characterized in that the N-substituted carbamoyl-lactam compound has the formula

$$R[-O-\underset{O}{\overset{}{C}}-\underset{H}{N}-R'-(\underset{H}{N}-\underset{O}{C}-\underset{\diagdown}{N}-\underset{\diagup}{C}=O)_a]_2 \quad (N\ \text{and}\ C\ \text{bridged by}\ Y)$$

wherein

R is a radical derived from polytetramethyleneglycol and represents the radical remaining from the polytetramethylene oxide starting material used in preparing polytetramethylene glycol and has an equivalent weight of 100-3000,

R' is an alkyl, cycloalkyl, aryl, alkaryl, or an aralkyl group, having 6-25 carbon atoms,

Y is a $C_3$-$C_{14}$ alkylene group, and

a has a value of at least 1.

A compound of this formula may be prepared by reacting an isocyanate terminated compound, prepared by reacting polytetramethyleneglycol and a polyisocyanate compound, with a lactam.

Advantageously the lactam can be caprolactam.

Surprisingly it has been found that nylon block copolymers prepared from these N-substituted carbamoyl-lactam compounds have superior properties compared with the nylon block copolymers prepared from N-substituted carbamoyl lactam compounds based on other diols such as polyethyleneglycol or polypropyleneglycol. These compounds can be used as an activator for the preparation of nylon blockcopolymers, in a combination with a lactam polymerization catalyst.

The polyol to be used in the present invention is polytetramethylene glycol (PTMG). The equivalent weight is advantageously 100-3000, more particularly about 300 to 2500. Most preferred is 1000-2500. It is also possible to use a mixture of polytetramethyleneglycol and one or more other polyoxyalkylene polyols of diols, triols or tetrols. These polyols can be of the same molecular weight as indicated above, but it is also possible to use one or more low molecular weight polyols such as glycerol or trimethylolpropane.

It is to be understood that any molecular weight, or equivalent weight, referred to herein are numerical average weights. Furthermore, the term equivalent weight of a polyol is understood to mean the numerical average weight of the polyol per hydroxyl group, i.e. the molecular weight divided by the functionality.

If a mixture of PTMG and at least one other polyol is used

in preparing the N-substituted carbamoyl-lactam compound, the OH-equivalent originating from the PTMG is at least 50 %, preferably between 60 and 95 %. This means that at least 50 % of all hydroxyl groups available for reaction with the polyisocyanate originate from PTMG.

Various polyisocyanates are suitable for use in the present process. Broadly speaking, such polyisocyanates having an isocyanate functionality of two or more include aliphatic, araliphatic, cycloaliphatic and aromatic isocyanates with 6-25 carbon atoms. Examples include 1,5 and 1,6 hexane diisocyanate, isophoronediisocyanate (IPDI), and xylylene diisocyanate (XDI). Readily available suitable di- and polyisocyanates include 2,4- and 2,6-toluene diisocyanate, 2,2'-,2,4'-, and 4,4'-diphenylmethane diisocyanate (MDI or crude MDI). Other polyisocyanates include polyphenylene polymethylene polyisocyanates. Also suitable polyisocyanates include modified MDI, and the hydrogenated aromatic diisocyanates such as hydrogenated TDI, XDI or MDI. Also suitable are the three or higher functional polyisocyanates, including the isocyanurate containing trimers of two or more diisocyanates. These isocyanates may be employed singly or in mixed form.

The value of a as given in the formula described hereinbefore, depends on the functionality of the isocyanate used.
The value of a is the mean functionality of the polyisocyanate (f) minus one (a = f-1).

Thus, if a substantially pure diisocyanate is used, the value of a is one. If, on the other hand, a three or higher functional polyisocyanate is used, such as an isocyanurate containing trimer, the value of a will be two or higher.

The invention is also directed to a process for preparing a lactam compound which consists essentially in mixing and adding a polyhydroxyl compound

$$R(OH)_2$$

wherein $R(OH)_2$ is polyetramethylene glycol, R is the radical remaining from the polytetramethylene oxide starting material used to prepare said polytetramethylene glycol, said polyhydroxyl compound having an equivalent weight of 300 to 2500, with a polyisocyanate

having the formula:

OCN-R'-NCO

wherein R' is an alkyl, cycloalkyl, aryl, alkaryl or an aralkyl group having 6-25 carbon atoms

and thereafter reacting the intermediate product thus formed with a lactam whereby there is obtained a lactam compound having the formula:

```
R[-O-C-N-R'-(N-C-N-C=O)a]2
     ‖ |      | ‖ \ /
     O H      H O  Y
```

wherein R and R' have the aforestated meanings and a is the functionality of said polyisocyanate minus 1.

The N-substituted carbamoyllactam compound is advantageously used as an activator in the preparation of nylon-block copolymers (anionic polymerization), by reactively admixing a lactam, such as caprolactam, a lactam polymerization catalyst such as sodium lactamate, potassium lactamate, or bromo magnesium lactamate, and the activator of the invention, which also constitutes an embodiment of the invention.

The amount of catalyst used can range from 0.01 to 10 equivalent %, with respect to lactam and more particularly between 0.10 and 2 equivalent % with respect to the lactam.

The amount of activator with respect to the final product lies advantageously between 5 and 35 wt.%, more particularly between 10 and 30 wt.% with respect to the final product.

Within these ranges the combination of impact strength and flexural modulus of the nylon block copolymer is optimal.

The anionic polymerization of lactam is advantageously conducted at a temperature between the melting point of the lactam and the melting point of the resulting nylon block copolymer. More particularly the polymerization is conducted at a temperature between 90 °C and 160 °C.

## EXAMPLES

### Example 1

Polymeg 1000 (polytetramethylene glycol having a molecular

weight of 1000, Quaker Oats Co.'s product) in an amount of 300 grams (0.60 equivalent) and hexamethylene diisocyanate in an amount of 104 grams (1.24 equivalent) were charged in a reaction kettle, and heated at 80 °C for one hour and 45 min. to obtain an isocyanate-terminated prepolymer having a NCO percent of 6.65. 72.9 grams (0.64 mole) of caprolactam was added to the isocyanate-terminated prepolymer and heated for 2 hours at 85 °C.

The resultant adduct was highly viscous at room temperature, and was liquid at 65 °C.

The adduct was copolymerized with caprolactam in the presence of Nyrim Catalyst Concentrate (a Grignard lactamate produced by Monsanto Company).

The rate study shown in Table I shows that the rate of copolymerization in terms of solidification time increases in accordance with the increasing amount of adduct in the copolymer.

Table I

Rate Study of HDI Adduct at 140 °C

| Product No. | Catalyst eq.% | Polyether wt.% | Solidification Time |
|---|---|---|---|
| 1 | 4.1 | 11.3 | 15 min. |
| 2 | 5.0 | 22.6 | 12 min. |
| 3 | 6.9 | 33.9 | 7 min. |

The adduct further was copolymerized with caprolactam in the presence of potassium lactamate. Tabel IA shows that the use of potassium lactamate gives a much faster solidification even at lower temperature than the Grignard catalyst in Tabel I.

Table IA

Rate Study of HDI Adduct at 130 °C

| Product No. | Catalyst eq.% | Polyether wt.% | Solidification Time |
|---|---|---|---|
| 13 | 2.1 | 10.2 | 2 min. 30 sec. |
| 14 | 3.1 | 10.2 | 1 " 55 " |
| 15 | 6.3 | 10.2 | 2 " 20 " |
| 16 | 3.3 | 20.2 | 2 " 40 " |
| 17 | 6.5 | 20.2 | 1 " 55 " |
| 18 | 7.0 | 30.3 | 5 " 25 " |

Example 2

Takenate 600 (hydrogenated xylylene diisocyanate, Takeda Chemical Ind. Co.'s product) ($H_6XDI$), in an amount of 120 grams (1.24 equivalent) and Polymeg 1000 (300 grams, 0.60 equivalent) were reacted at 75 °C for 2.2. hours. Into the reaction product, 72.3 gram of caprolactam was added and heated for 2 hours at 85 °C.

Examples of the rate of copolymerization using lactam magnesium-bromide as catalyst at 140 °C are shown in Table II, product no. 4 , 5 and 6.

Examples of the rate of copolymerization using potassium lactamate as catalyst at 130 °C are shown in Table II, product no. 19, 20 and 21.

Table II

Rate Study of $H_6XDI$-Polymeg 1000 adduct

| Product No. | Polyether (wt.%) | Catalyst (eq.%) | Solidification Time |
|---|---|---|---|
| 4 | 32.8 | 6.5 | 11 min. |
| 5 | 21.9 | 5.0 | 16 min. |

| Product No. | Polyether (wt.%) | Catalyst (eq.%) | Solidification Time |
|---|---|---|---|
| 6 | 10.9 | 4.1 | 20 min. |
| 19 | 20.1 | 2.2 | 1 min. 50 sec. |
| 20 | 20.1 | 3.2 | 1 min. 55 sec. |
| 21 | 20.1 | 6.5 | 1 min. 30 sec. |

Example 3

Isonate 143L (a liquified diphenylmethane diisocyanate, isocyanate equivalent: 144, Upjohn's product) in an amount of 177 grams was charged in a reaction kettle.

300 grams of Polymeg 1000 was mixed with 50 grams of dioxane, and then the dioxane solution was added drop-by-drop into the Isonate 143L in the reaction kettle.

The reaction temperature was elevated slowly up to 80 °C.

The reaction time was noted as one hour and 35 minutes.

The stoichiometric amount of caprolactam (71.3 grams) was then charged into the prepolymer and reacted to obtain a caprolactam-terminated adduct.

The solvent, dioxane, was removed under vacuum after the adduct was prepared.

Some of the copolymerizations of the adduct with caprolactam using lactam magnesiumbromide as a catalyst, are shown in Table III.

Table III

Reaction Rate of MDI Adduct (1:2) and
MDI Adduct (1 : 3) Mole at 140 °C

| Product No. | Activator eq.% | Polyether (wt.%) | Catalyst (eq.%) | Cloud Point | Solidification 1:2 Mole | 1:3 Mole |
|---|---|---|---|---|---|---|
| 7 | 13.7 | 29.3 | 6.6 | * | 18 Min. | 70 Min. |
| 8 | 7.0 | 19.6 | 5.1 | * | 19 Min. | 35 Min. |
| 9 | 2.8 | 9.8 | 4.1 | 16 Min. | 24 Min. | 10 Min. |

* After Solidification Time

Example 4

A TDI-terminated Polymeg 1000 (polytetramethylene ether diol having a molecular weight of 1000) was prepared at 2:1 molar ratio in the presence of 0.1 % of T-9 catalyst. The reaction mixture was heated at 80 °C for 1.5 hours to reach nearly theoretical NCO percent.

Then, a stoichiometrical amount of caprolactam was added to the TDI-terminated prepolymer, and the mixture was reacted at 80 °C for 2 hours.

The reaction mixture was almost solid at room temperature.

The caprolactam adduct of TDI-terminated urethane prepolymer did not completely copolymerize with caprolactam, and the reaction product after one hour was a highly viscous liquid, and was not solid.

Example 5

(a) Preparation of an MDI adduct. A MDI-terminated urethane prepolymer was prepared by using Polymeg 1000 and Isonate 1431 (a liquified MDI).

In the case of 2:1 molar ratio, the resulting prepolymer was highly viscous and was very difficult to handle.

Therefore, the molar ratio was changed to 3:1. Accordingly, the reaction product contains also MDI-caprolactam adduct in low molecular weight. The preparation procedures employed were almost the same as those employed in Example 4 with the TDI prepolymer.

The thus obtained adduct was solid at room temperature and melted at about 90 °C.

(b) Copolymerization of MDI Adduct. The adduct composed of MDI-terminated Polymeg 1000-based urethane prepolymer (25 parts by weight), caprolactam (75 parts by weight) and a 1 molar solution of lactam magnesiumbromide in caprolactam (20 parts by weight) were mixed in a beaker at 145 °C for 10 min., and its viscosity was increased to sufficient level. The reactant was then transferred to a mold kepts at 145 °C. After one minute and 30 seconds, the reacting mass became a gel. After 5 minutes mold retention, the polymerized mass was demolded and its physical properties were measured. The physical properties are listed in Table IV.

Table IV

Physical Properties of Copolymer Composed of Caprolactam and Caprolactam Adduct of MDI-terminated Polymeg 1000-based Urethane Prepolymer

| Physical Characteristic | Property |
|---|---|
| Shore D Hardness | 77 |
| Tensile Strength, psi. | 5220 |
| Elongation, % | 372 |
| Tear Strength, pli | 1440 |
| Notched Izod Impact, ft.lbs/in | 7.5 |
| Water Absorption, 23 °C | |
| 24 hours, % | 4.28 |
| 168 hours, % | 8.69 |
| Decomposition Temperature °C by TGA | |
| 2 % decomposition | 225 |
| 10 % decomposition | 310 |
| 50 % decomposition | 370 |

Example 6

Copolymerization of caprolactam adduct of MDI-terminated Polymeg 2000-based urethane prepolymer prepared according to example 5, with caprolactam was conducted by using lactam magnesiumbromide as catalyst.

Preparation of NCO-terminated Prepolymers Using MDI and Polymeg 2000.

250 grams of Polymeg 2000 demoisturized under vacuum was reacted with 108 grams of MDI (Isonate 143L) in the presence of 0.1 % of T-9 (a tin catalyst) at 80 °C. It took 75 min. to reach to the theoretical NCO content.

The product was very viscous at 80 °C, and was very difficult to stir.

Therefore, a prepolymer was prepared without the use of a tin catalyst in a molar ratio of MDI/Polymeg 2000 = 2/1 and in the presence of 10 grams of dioxane as a solvent.

The reaction was completed in 135 min. at 80 °C.

Into the prepolymer, stoichiometrical amount of caprolactam was added and stirred at 80 °C for 2 hours to obtain a caprolactam adduct. The reaction product was then heated to 110 °C to remove dioxane at atmospheric pressure, followed by vacuum distillation to remove the last traces of dioxane.

The copolymerization of the Polymeg 2000 adduct with caprolactam was conducted by using the same manner employed in the previous examples.

The rate of copolymerization of the above adduct with caprolactam is shown in Table V.

Some of the physical properties of the copolymer are shown in Table VI.

Table V

Copolymerization of Caprolactam Adduct of MDI-terminated Polymeg 2000-based Urethane Prepolymer at 145 °C

| Product No. | Activator eq.% | Polyether (wt.%) | Catalyst (eq.%) | Cloud Point Min. | Solidification Time, min. |
|---|---|---|---|---|---|
| 10 | 1.8 | 12.7 | 4.1 | 19 | 28 |
| 11 | 4.5 | 25.3 | 5.2 | 14 | 22 |
| 12 | 9.1 | 38.3 | 7.0 | 4 | 12 |

Table VI

Properties of Polymeg 2000-based Nylon 6 Copolymer at 145 °C

| Product No. | Tensil Strength psi | 100 % Modulus psi | Elongation % | Tear Strength pli | Shore D |
|---|---|---|---|---|---|
| 10 | 2103 | 1506 | 342 | 345 | 48 |
| 11 | 4394 | 1540 | 490 | 637 | 57 |
| 12 | 1420 | 472 | 354 | 185 | 38 |

Example 7

250 g of dry Polymeg 2000 was reacted at 80 °C with 42 grams hexamethylene diisocyanate (HDI) in the presence of 10 grams of dioxane. To the prepolymer a stoichiometrical amount of caprolactam was added and the mixture obtained was stirred at 80 °C for 2 hours to obtain a caprolactam adduct. After evaporation of the dioxane, 55.6 grams of the adduct and 40 grams caprolactam were heated at 95 °C (solution A). Seperately a solution of 19.5 g lactam magnesiumbromide (1 molar in caprolactam) in 60 g caprolactam was prepared at 95 °C (solution B). Solution A and B were mixed together and poured into a mold maintained at 160 °C. After 15 minutes at 160 °C the sample was demolded. The obtained nylon copolymer (25 wt.% polyether) showed a flexural modulus of 1129 MPa and a notched Izod Impact strength of 4.9 ft. lbs/in.

WE CLAIM:

1. An N-substituted carbamoyl-lactam compound of the formula

```
R[-O-C-N-R'-(N-C-N-C=O)a]2
    || |     | || \ /
    O  H     H O   Y
```

wherein R is a radical derived from polytetramethyleneglycol $R(OH)_2$ and represents the radical remaining from the polytetramethylene oxide starting material used in preparing polytetramethyleneglycol and has an equivalent weight of 300-2500,

R' is an alkyl, cycloalkyl, aryl, alkaryl or an aralkyl group having 6-25 carbon atoms,

Y is a $C_3$-$C_{14}$ alkylene group, and

a has a value of at least 1.

2. Compound according to claim 1, characterized in that R' is a radical derived from a polyisocyanate selected from the group consisting of 1,5 hexane diisocyanate, 1,6 hexane diisocyanate, isophoronediisocyanate, xylylene diisocyanate, 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, polyphenylene polymethylene polyisocyanates, hydrogenated 2,2'-diphenylmethane diisocyanate, hydrogeneted 2,4'-diphenylmethane diisocyanate, hydrogenated 4,4'-diphenylmethane diisocyanate, hydrogenated xylylene diisocyanate, hydrogenated 2,4-toluene diisocyanate, and hydrogenated 2,6-toluene diisocyanate and mixtures thereof.

3. Compound according to claim 2, characterized in that said polyisocyanate is hexamethylene diisocyanate, isophorone diisocyanate, TDI or MDI.

4. Compound according to claim 1, wherein $R(OH)_2$ is the mixture of polytetramethylene glycol and at least one other polyol.

5. Compound according to claim 1 substantially as described and elucidated in the examples.

6. A process for preparing a lactam compound which consists essentially in mixing and adding a polyhydroxyl compound

$$R(OH)_2$$

wherein $R(OH)_2$ is polyetramethylene glycol, R is the radical remaining from the polytetramethylene oxide starting material used to prepare said polytetramethylene glycol, said polyhydroxyl compound having an equivalent weight of 300 to 2500, with a polyisocyanate having the formula:

$$OCN-R'-NCO$$

wherein R' is an alkyl, cycloalkyl, aryl, alkaryl or an aralkyl group having 6-25 carbon atoms;

and thereafter reacting the intermediate product thus formed with a lactam whereby there is obtained a lactam compound having the formula:

```
R[-O-C-N-R'-(N-C-N-C=O)a]2
      ||  |      |  ||  \_/
      O   H      H  O    Y
```

wherein R and R' have the aforestated meanings and a is the functionality of said polyisocyanate minus 1.

7. A process according to claim 6 characterized in that the polyisocyanate is selected from the group consisting of 1,5 hexane diisocyanate, 1,6 hexane diisocyanate, isophoronediisocyanate, xylylene diisocyanate, 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, polyphenylene polymethylene polyisocyanates, hydrogenated 2,2'-diphenylmethane diisocyanate.

8. Process according to claim 7, characterized in that said polyisocyanate is hexamethylene diisocyanate, isophorone diisocyanate, TDI or MDI.

9. Process according to claim 6, characterized in that as lactam caprolactam is used.

10. Process according to claim 6, substantially as described and elucidated in the examples.

11. Process for preparing a nylon blockcopolymer by polymerizing lactam in the presence of a lactam polymerization catalyst and an N-substituted carbamoyllactam compound of claim 1.

European Patent Office

# EUROPEAN SEARCH REPORT

0159070
Application number

EP 85 20 0438

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 511 893 (P.R. SCHAEFFER et al.)<br>* Claim 1; examples 2,3 *<br>--- | 1,5,6, 9-11 | C 08 G 18/10<br>C 08 G 18/48<br>C 08 G 69/20 |
| X | DIE ANGEWANDTE MAKROMOLEKULARE CHEMIE, vol. 58/59, no. 844, February 1977, pages 321-343, Basel, CH; W.T. ALLEN et al.: "Caprolactam based block copolymers using polymeric activators"<br>* Summary; page 325, last paragraph - page 328, paragraph 2; page 323, paragraph 4 *<br>--- | 1-11 | |
| A | US-A-3 245 961 (C.A. FETSCHER et al.)<br>* Claim 1; column 3, line 48 - column 4, line 50 *<br>--- | 1 | **TECHNICAL FIELDS SEARCHED (Int. Cl.4)** |
| A | GB-A-1 099 265 (BRITISCH CELANESE)<br>--- | | C 08 G |
| D,A | GB-A-1 067 153 (ICI)<br>----- | | |

The present search report has been drawn up for all claims

| Place of search | Date of completion of the search | Examiner |
|---|---|---|
| THE HAGUE | 29-07-1985 | VAN PUYMBROECK M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82